# EUROPÄISCHE PATENTSCHRIFT

(11) **EP 1 254 099 B1**
(45) Veröffentlichungstag und Bekanntmachung des Hinweises auf die Patenterteilung: **26.04.2006**
(21) Anmeldenummer: 01901198.0
(22) Anmeldetag: 24.01.2001
(51) Int. Cl.: C07C 53/02, C22C 38/44, C22C 19/05, C22C 14/00

(54) **VERFAHREN ZUR HERSTELLUNG VON WASSERFREIER AMEISENSÄURE**
PROCESS FOR THE PRODUCTION OF ANHYDROUS FORMIC ACID
PROCEDE POUR LA PRODUCTION D'ACIDE FORMIQUE ANHYDRE

(30) Priorität: 24.01.2000 DE 10002795
(43) Veröffentlichungstag der Anmeldung: 06.11.2002
(73) Patentinhaber: BASF Aktiengesellschaft, 67056 Ludwigshafen (DE)
(72) Erfinder: AUER, Heinz, 68809 Neulussheim (DE); BESSLING, Bernd, 67269 Grünstadt (DE); HAMMER, Hans, 68219 Mannheim (DE); HASSE, Hans, 67661 Kaiserslautern (DE); SAUER, Friedrich, 67271 Obersülzen (DE); VICARI, Maximilian, 67117 Limburgerhof (DE); WAGNER, Gerhard, 67069 Ludwigshafen (DE); ADRIAN, Till, 67240 Bobenheim-Roxheim (DE)
(74) Vertreter: Isenbruck, Günter
(86) Internationale Anmeldenummer: PCT/EP2001/000748
(87) Internationale Veröffentlichungsnummer: WO 2001/055077

(56) Entgegenhaltungen:
- EP-A- 0 717 028
- DE-A- 2 513 678
- US-A- 4 008 344
- US-A- 4 415 532
- US-A- 5 338 508
- GASSEN R ET AL: "KORROSION IN CARBONSAEUREN TEIL II: DAS KORROSIONSVERHALTEN HOCHLEGIERTER STAEHLE, ZWEIER NICKELLEGIERUNGEN UND VON TITAN IN AMEISENSAEURE CORROSION IN CARBOXYLIC ACIDS - PART II: THE CORROSION BEHAVIOR OF STAINLESS STEELS, NICKEL ALLOYS AND TITANIUM IN FORMIC ACID" , ZEITSCHRIFT FUER WERKSTOFFTECHNIK - JOURNAL OF MATERIALS TECHNOLOGY. MATERIALS TECHNOLOGY AND TESTING,VCH, WEINHEIM,DE, VOL. 17, NR. 6, PAGE(S) 218-225 XP000995972 ISSN: 0049-8688 Stähle 2.4610 bzw. 2.4858 Tabelle 1
- SCHILLMOLLER C M: "CONTROL ORGANIC-ACID CORROSION WITH THESE METALS AND ALLOYS" , CHEMICAL ENGINEERING PROGRESS,AMERICAN INSTITUTE OF CHEMICAL ENGINEERS,,US, VOL. 93, NR. 2, PAGE(S) 66-71 XP000995964 ISSN: 0360-7275 Seite 68, Absatz "Formic acid", insbesonders letzter Absatz Tabelle 1
- CHEMICAL ABSTRACTS, vol. 107, no. 12, 21. September 1987 (1987-09-21) Columbus, Ohio, US; abstract no. 104908, ZARITSKII, V. D. ET AL: "Corrosion of alloy KhN40MDB in formic acid-water system" XP002170575 -& ZASHCH. MET. (1987), 23(3), 481-3 , XP002170574
- CHEMICAL ABSTRACTS, vol. 105, no. 26, 29. Dezember 1986 (1986-12-29) Columbus, Ohio, US; abstract no. 228902, KAJAMA, HIROHISA ET AL: "Handling of carboxylic acids" XP002170576 -& JP 53 127408 A (TOKUYAMA SODA CO., LTD., JAPAN) 7. November 1978 (1978-11-07)

## Beschreibung

Die Erfindung betrifft ein Verfahren zur Gewinnung von wasserfreier oder weitgehend wasserfreier Ameisensäure wird den Einsatz bestimmter Werkstoffklassen als Konstruktionsmaterialien in der Anlage, in der des Verfahren durchgeführt wird.

Aus der EP-B-0 017 866 geht hervor, daß man wasserfreie oder weitgehend wasserfreie Ameisensäure erhält, wenn man
a) Methylformiat der Hydrolyse unterwirfr,
b) vom erhaltenen Hydrolysegemisch Methanol sowie überschüssiges Methylformiat abdestilliert, ,
c) das Ameisensäure und Wasser enthaltende Sumpfprodukt der Destillation b) in einer Flüssig-flüssig-Extraktion mit einem hauptsächlich die Ameisensäure aufnehmendem Extraktionsmittel extrahiert,
d) die hierbei erhaltene Ameisensäure und eine Teilmenge des Wassers aufweisende Extraktphase einer Destillation unterwirft,
e) das bei dieser Destillation erhältliche, Wasser und eine Teilmenge der Ameisensäure enthaltende Kopfprodukt in den unteren Teil einer Destillationseinrichtung der Stufe b) zurückführt,
f) das vorwiegend Extraktionsmittel und Ameisensäure enthaltende Sumpfprodukt der Destillationsstufe d) destillativ in wasserfreie oder weitgehend wasserfreie Ameisensäure und das Extraktionsmittel auftrennt und
g) das die Stufe f) verlassende Extraktionsmittel in den Verfahrensgang zurückführt

Zur Durchführung dieses Verfahrens wird dann eine Vorrichtung bereitgestellt, die folgende Elemente enthält,
- einen Synthesereaktor zur Herstellung von Methylformiat,
- einen Hydrolysereaktor zur Hydrolyse von Methylformiat,
- eine Destillationseinrichtung - zur Abtrennung von überschüssigem Methanol sowie überschüssigem Methylformiat von dem Hydrolysegemisch (Stufe b)),
- eine Destillationseinrichtung zur Destillation einer Ameisensäure, Extraktionsmittel und eine Teilmenge des Wassers aufweisenden Extraktphase (Stufe d)),
- eine Extraktionseinrichtung zur Durchführung der Stufe c) und
- eine Destillationseinrichtung zur destillativen Auftrennung eines vorwiegend Extraktionsmittel und Ameisensäure enthaltenden Gemischs in wasserfreie oder weitgehend wasserfreie Ameisensäure und Extraktionsmittel (Stufe f)).

Die entsprechenden Elemente der Vorrichtung, die mit Ameisensäure kontaktieren, müssen unbedingt aus Werkstoffen konstruiert sein, die korrosionsbeständig sind. Ameisensäure, insbesondere wäßrige Ameisensäure ist ein äußerst starkes Korrosionsmittel. Unter Korrosion soll die Wechselwirkung eines Werkstoffs mit seiner Umgebung verstanden werden, die eine meßbare Veränderung des Werkstoffs bewirkt und zu einer Beeinträchtigung der Funktion des entsprechenden Bauteils oder des ganzen Systems führt. Korrosion soll somit als die von der Oberfläche ausgehende, durch unbeabsichtigte chemische oder elektrochemische Angriffe hervorgerufene, nachteilige und qualitätsmindernde Veränderung eines Werkstoffes verstanden werden. Das angreifende Mittel, das chemisch oder elektrochemisch wirkt, wird als korrosives Mittel bezeichnet - Ameisensäure, insbesondere wäßrige Ameisensäure wird als stark korrosives Mittel angesehen.

Anlagen zur Herstellung von Ameisensäure bzw. wasserfreier Ameisensäure müssen daher in besonderem Maße vor Korrosion geschützt werden. Dabei eignen sich nur sehr wenige Werkstoffe als Konstruktionsmaterial für die mit Ameisensäure kontaktierenden Anlagenteile. In der Literatur [T.-L. Yau, K. W. Bird, Chemical Engineerging Process, January 1992, Seite 65], [T.-L. Yau, K. W. Bird, Chemical Engineerging Process, January 1995, Seite 42], [Firmeninformation: "Outlook", Teledyne Vah Chang Albany, Winter/Spring 1990 Band 11, Nr. 1, Seiten 1-3] wird dafür ausschließlich Zirkonium und Zirkonium-Legierungen, welche einen Anteil von mindestens 90 % Zirkonium aufweisen, als Werkstoffe empfohlen. Zirkonium wird beim Kontaktieren mit Ameisensäure passiviert - bildet eine stabile oxidische Schutzschicht. Andere Materialien eignen sich weniger als Werkstoff - bzw. geeignete Materialien (außer Zirkonium) sind so teuer, daß diese nicht wirtschaftlich einsetzbar sind. Somit werden in der Technik Anlagenteile, die mit Ameisensäure kontaktieren, in der Regel ausschließlich aus Zirkonium oder Zirkonium-Legierungen, welche einen Anteil von mindestens 90 % Zirkonium aufweisen, hergestellt. Nachteilig ist, daß zirkoniumreiche Werkstoffe teuer sind und daher die Investitionskosten für entsprechende Anlagenteile hoch sind.

Aus dem Stand der Technik sind auch noch die folgenden Dokumente bekannt.

In der BP-A 0 717 028 ist in etwa ein mit der vorstehenden EP-B vergleichbares Verfahren bekannt. Gassen R. et al. in "Korrosion in Carbonsäujren, Teil II", Zeitschrift für Werkstofftechnik, VCH-Weinheim, Vol. 17, 6, S. 218-225, untersuchen das Korrosionsverhalten von Stählen, Nickellegierungen und Titan gegenüber Ameisensäure.

In der US 4,415,532 werden Legierungen in ihrem Verhalten gegenüber Ameisensäure bei niedrigerer Temperatur untersucht, deren Ni-Gehalt geringer und deren C-Gehalt höher ist.

Aus Schillmüner C.M. "Control organic acid corrosion with these metals and alloys", Chem. Eng. Progress, American Institute of Chemical Engineers, Vol. 93, 2, S. 66-71 ist bekannt, im LabormaBstab bei Temperaturen unter 100°C zu arbeiten.

Die US 5,338,508 beschreibt Korrosionsversuch in Gemischen aus Essig- und Ameisensäure. Zaritskii V.D. et al. in Chemical Abstracts, Vol. 107, No. 12, 1987, Abstract No. 104908 untersuchen eine sehr spezielle Legierung (KhN40MDB-VI) in Behältern mit PTFE-Auskleidung.

Kajama Hirohisa et al. in Chemical Abstracts, Vol. 105, No. 26, 1986, Abstract No. 228902 untersuchen Stähle mit einem Cr-Gehalt über 21% und einem Ni-Gehalt von 0,1 bis 20%v.

Database Compendez, Abstract No. EIX 91110665032 untersucht das Korrosionsverhalten von Titan-Legierungen. Ebenfalls wird mit Titan- und PD-Legierungen beschäftigt sich auch Chemical Engineering Progress 88, No. 2, Februar 1992, S. 65-69.

Aufgabe der vorliegenden Erfindung ist e5, ein Verfahren bereitzustellen, mit der das vorstehend genannte Verfahren zur Herstellung von wasserfreier bzw. weitgehend wasserfreier Ameisensäure durchgeführt werden kann und die Materialkosten für die Konstruktion einer geeigneten Anlage niedriger sind als vergleichsweise für eine entsprechende Anlage, die aus zirkoniumreichen Werkstoffen konstruiert ist.

bei dem man in einer Anlage,
α) Methylformiat der Hydrolyse unterwirft,
β) vom erhaltenen Hydrolysegemisch Methanol sowie überschüssiges Methylformiat abdestilliert,
χ) das Ameisensäure und Wasser enthaltende Sumpfprodukt der Destillation (β) in einer nossig-Sussig-Extraktion mit einem hauptsächlich die Ameisensäure aufnehmenden Extraktionsmittel extrahiert und dabei als Extraktionsmittel ein Carbonsäureamid der allgemeinen Formel 1 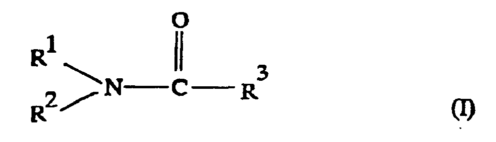 einsetzt, in der die Reste R¹ und R² Alkyl-, Cycloalkyl-, Aryl- oder Aralkylgruppen bedeuten oder R¹ und R² gemeinsam zusammen mit dem N-Atom einen heterocyclischen 5- oder 6-Ring ausbilden und in der nur einer der Reste eine Arylgruppe ist und in der R³ für Wasserstoff oder eine C₁-C₄-Alkylgruppe steht,
δ) die hierbei erhaltene Ameisensäure, Extraktionsmittel und eine Teilmenge des Wassers aufweisende Extraktphase einer Destillation unterwirft,
ε) das bei dieser Destillation erhaltene Wasser und eine Teilmenge der Ameisensäure enthaltende Kopfprodukt in den unteren Teil der Destillationseinrichtung zur Durchführung der Stufe β) zurückführt.
φ) das vorwiegend Extraktionsmittel und Ameisenäure enthaltende Sumpfprodukt der Destillationsstufe δ) destillativ in wasserfreie oder weitgehend wasserfreie Ameisensäure und das Extraktionsmittel auftrennt und
γ) das die Stufe φ) verlassende Extraktionsmittel in den Verfahrensgang zurückführt.

Das erfindungsgemäße Verfahren ist dann dadurch gekennzeichnet, daß ein oder mehrere Anlagenteile, die mit Ameisensäure und mit Extraktionsmittel kontaktieren, teilweise oder vollständig aus einem zirkoniumarmen Werkstoff konstruiert sind, der ausgewählt ist aus der Gruppe der Werkstoffklassen. Titan-Palladinm-Legierungen, chrom-, molybdän- und/oder wolframhaltige Nickelbasiswerkstoffe und molybdänhaltige hochlegierte austenitische Chrom-Nickel-Sonderstähle.
- die chrom-, molybdän- und/oder wolframhaltigen Nickelbasiswerkstoffe 14 bis 24 Gew.-% CT, 8 bis 17 Gew.-% Mo und/oder 3 bis 5 Gew.-% W, maximal 25 Gew.-% sonstige Elemente und den Rest als Ni enthalten,
- die molybdänhaltigen hochlegierten austenitischen Chrom-Nickel-Sonderstähle 18 bis 30 Gew.-% Cr, 12 bis 40 Gew.-% Ni, 3 bis 7 Gew.-% Mo, maximal 3 Gew.-% Cu, maximal 0,05 Gew.-% C, maximal 25 Gew.-% sonstige Elemente und den Rest als Fe enthalten, und
- die Temperaturen im Verfahren 100°C bis 190°C betragen, und wobei die mit Ameisensäure und mit Extraktionsmittel kontaktierenden Anlagenteile als Reaktor zur Durchführung der Stufe α), als Destillationseinrichtung zur Durchführung der Stufe β), als Destillationseinrichtung zur Durchführung der Stufe δ), als Extraktionseinrichtung zur Durchführung der Stufe χ) und/oder als Destillationseinrichtung zur Durchführung der Stufe φ), vorliegen.

Diese beiden Gruppen von Werkstoffen sind erfindungswesentlich.

Beispiele für Titan-Palladium-Legierungen sind Legierungen, welche 0,1 bis 0,25 Gew.-% Pd, maximal 0,4 Gew.-% Sauerstoff, maximal 0,5 Gew.-% Fe sowie maximal je 1 Gew.-% an sonstigen Elementen und den Rest als Titan enthalten, wobei diese sonstigen Elemente - übliche herstellungsbedingte Beimengungen und Verunreinigungen - insgesamt maximal 2 Gew.-% betragen - bezogen auf die Gesamtzusammensetzung. Titan-Palladium-Legierungen dieser Zusammensetzung sind in dieser Werkstoffklasse bevorzugt.

Sofern die genannten Legierungen Entsprechungen anderer Bezeichnung haben, sollen diese bei vergleichbarer Zusammensetzung und Eigenschaft mit umfaßt sein.

Unter weitgehend wasserfreier Ameisensäure soll Ameisensäure verstanden werden, die maximal 30 Gew.%, bevorzugt maximal 15 Gew.% Wasser enthält. Als Synthesereaktor soll eine Einrichtung verstanden werden, in der einerseits die Synthese von Methylformiat erfolgt (meist in einem entsprechenden Reaktor) und gegebenenfalls andererseits noch eine Trennung des erhaltenen Synthesegemischs (meist in einer dem Reaktor nachgeschalteten Destillationseinrichtung) durchgefiihrt wird. Als Hydrolysereaktor eignet sich jeder beliebige Reaktor, der fiir die Hydrolyse von Methylformiat eingesetzt werden kann. Bevorzugt wird als Extraktionseinrichtung eine Flüssig-flüssig-Extraktionskolonne eingesetzt. Als Destillationseinrichtungen kommen insbesondere Destillationskolonnen in Frage.

In den nachstehenden Tabellen sind in Frage kommende Werkstoffklassen (Klassen I bis III) näher definiert. Unter sonstigen Elementen versteht man sowohl gezielt zugesetzte weitere Elemente als auch übliche herstellungsbedingte Beimengungen und Verunreinigungen.

**Tabelle 1**

| Klasse I | Klasse II | | Klasse III | |
|---|---|---|---|---|
| Titan-Palladium-Legierungen | chrom-, molybdän- und/oder wolframhaltige Nickelbasiswerkstoffe | | molybdänhaltige hochlegierte austenitische Chrom-Nickel-Sonderstähle | |

**Tabelle 2**

| | | | | |
|---|---|---|---|---|
| | Klasse I | | | |
| | Titan-Palladium-Legierungen | | | |
| Charakteristische Legierungszusammensetzung (Hauptelemente) | Palladium: 0,1- 0,25 Gew.% | | | |
| | Sauerstoff maximal 0,4 Gew.% | | | |
| | Eisen: maximal 0,5 Gew.% | | | |
| | Sonstige Elemente: einzeln jeweils maximal 1 Gew.%, insgesamt maximal 2 Gew.-%; entsprechend den unten genannten Werkstoffen/Normen | | | |
| | Rest (Basiswerkstoff): Titan | | | |

| | Land | Kurzbezeichnung | Werkstoffnr | Norm |
|---|---|---|---|---|
| Beispiele | D | Ti1Pd | 3.7225 | DIN 17851 |
| | | Ti2Pd | 3.7235 | DIN 17851 |
| | | Ti3Pd | 3.7255 | DIN 17851 |
| | USA | Ti Pd grade 7 | UNS R52400 | ASTM B265, B337, B338, B348, B381 |
| | | Ti Pd grade 11 | UNS R52250 | ASTM B265, B337, B338, B348, B381 |

**Tabelle 3**

| | | | | |
|---|---|---|---|---|
| | Klasse II | | | |
| | chrom-, molybdän- und/oder wolframhaltige Nickelbasiswerkstoffe | | | |
| Charakteristische Legierungszusammensetzung (Hauptelemente) | Chrom: 14-24 Gew.% | | | |
| | Molybdän: 8-17 Gew.% und/oder Wolfram: 3 - 5 Gew.% | | | |
| | Sonstige Elemente: einzeln jeweils maximal 10 Gew.-%, insgesamt maximal 25 Gew.-%; entsprechend den unten genannten Beispielen | | | |
| | Rest (Basiswerkstoff): Nickel | | | |

| | Land | Kurzbezeichnung | Werkstoffnr | Norm |
|---|---|---|---|---|
| Beispiele | D | NiMo16Cr15W | 2.4819 . | DIN 17744 |
| | USA | Alloy C-276 | UNS N10276 | ASTM B366, B564, B574, B575, B619, B622, B626 |
| | D | NiCr22Mo9Nb | 2.4856 | DIN 17744, EN 10095 |
| | USA | Alloy 625 | UNS N06625 | ASTM B366, B443, B444, B446, B704, B 705 |
| | D | NiMo16Cr16Ti | 2.4610 | DIN 17744 |
| | USA | Alloy-C4 | UNS N06455 | ASTM B 574, B575, B619, B622, B626 |
| | D | NiCr23Mo16A1 | 2.4605 | VdTÜV-Werkstoffblatt 505 |
| | USA | Alloy 59 | UNS N06059 | ASTM B622, B619, B626, B575, B574, B564 |
| | D | NiCr21Mo16W | 2.4606 | VdTÜV-Werkstoffblatt 515 |
| | USA | Alloy 686 | UNS N06686 | ASTM B564, B574, B575, B619, B622, B626 |
| | D | NiCr23Mo16Cu | 2.4606 | Noch nicht genormt |
| | USA | Alloy C2000 (Hastelloy® C-2000® alloy) | | Noch nicht genormt |

**Tabelle 4**

| | | | | |
|---|---|---|---|---|
| | Klasse III | | | |
| | molybdänhaltige hochlegierte austenitische Chrom-Nickel-Sonderstähle | | | |
| Charakteristische Legierungszusammensetzung (Hauptelemente) | Chrom: 18-30 Gew.-% | | | |
| | Nickel: 12-40 Gew.-% | | | |
| | Molybdän:3-7 Gew.-% Molybdän: | | | |
| | Kupfer: maximal 3 Gew.-% | | | |
| | Kohlenstoff: maximal 0,05 Gew.-% | | | |
| | Sonstige Elemente: einzeln jeweils maximal 10 Gew.-%, insgesamt maximal 25 Gew.-%; entsprechend den unten genannten Beispielen | | | |
| | Rest (Basiswerkstoff): Eisen | | | |

| Beispiele | Land | Kurzbezeichnung | Werkstoffnr | Norm |
|---|---|---|---|---|
| | D | X2NiCrMoN 17-13-5 | 1.4439 | EN 10088-1-2-3, DIN 17440, DIN 17441 |
| | D | X1NiCrMoCuN 25-20-7 | 1.4529 | EN 10088-1-2-3 |
| | D | XINiCrMoCu 25-20-5 | 1.4539 | EN 10088-1-2-3 |
| | USA | | UNS N08904 | ASTM A240, A480, B625, B649, B673, B674, B677 |
| | D | X1CrNiMoCuN 20-18-7 | 1.4547 | EN 10088-1-2-3 |
| | USA | | UNS S31254 | ASTM A182, A 193, A194, A204, A249, A269, A276, A312, A358, A403, A409, A479, A813, A814 |
| | USA | | UNS S31725 | ASTM A167, A182, A213, A240, A249, A269, A276, A312, A358, A376, A409, A479 |
| | USA | | UNS S31726 | ASTM A167, A182, A213, A240, A249, A269, A276, A312, A358, A376, A409, A479 |

| | | | | |
|---|---|---|---|---|
| Genaue Konzentrationsangaben zu Beispielen aus den drei angeführten Werkstoffldassen | | | | |

**Tabelle 5 Klasse 1: Werkstoffnummer 3.7235**

| Chemische Zusammensetzung: Massenanteile in Gew.-% | | | | | | | | | |
|---|---|---|---|---|---|---|---|---|---|
| | | | | | | | Sonstige | | |
| | Fe | O | N | C | H | Pd | Einzeln | zusammen | Ti |
| Min | | | | | | 0,15 | | | Basis |
| Max | 0,20 | 0,18 | 0,05 | 0,06 | 0,013 | 0,25 | 0,1 | 0,4 | Basis |

**Tabelle 6 Klasse 2: Werkstoffnummer 2.4819**

| Chemische Zusammensetzung: Massenanteile in Gew.-% | | | | | | | | | | | |
|---|---|---|---|---|---|---|---|---|---|---|---|
| | | | | | | | | | Sonstige (V, Cu, W, Fe etc) | | |
| | C | Si | Mn | P | S | Co | Cr | Mo | einzeln | zusammen | Ni |
| Min | | | | | | | 14,5 | 15,0 | | | Basis |
| Max | 0,015 | 0,08 | 1,0 | 0,025 | 0,015 | 2,5 | 16,5 | 17,0 | 7,0 | 13,0 | |

**Tabelle 7 Klasse 2: Werkstoffnummer 2.4856**

| Chemische Zusammensetzung: Massenanteile in Gew.-% | | | | | | | | | | | | |
|---|---|---|---|---|---|---|---|---|---|---|---|---|
| | | | | | | | | | | Sonstige (Cu, Nb, Ti, Fe etc) | | |
| | C | Si | Mn | P | S | Al | Co | Cr | Mo | einzeln | Zusammen | Ni |
| Min | 0,03 | | | | | | | 20,0 | 8,0 | | | Basis (58,0) |
| Max | 0,10 | 0,5 | 0,5 | 0,020 | 0,015 | 0,40 | 1,0 | 23,0 | 10,0 | 5,0 | 10,0 | |

**Tabelle 8 Klasse 3: Werkstoffnummer 1.4439**

| Chemische Zusammensetzung: Massenanteile in Gew.-% | | | | | | | | | | |
|---|---|---|---|---|---|---|---|---|---|---|
| | | | | | | | | | | |
| | C | Si | Mn | P | S | N | Cr | Mo | Ni | Fe |
| Min | | | | | | 0,12 | 16,5 | 4,0 | 12,5 | Basis |
| Max | 0,030 | 1,0 | 2,0 | 0,045 | 0,015 | 0,22 | 18,5 | 5,0 | 14,5 | |

Das eingesetzte Extraktionsmittel wirkt bei Kontaktierung der vorstehend genannten Werkstoffklassen mit wäßriger Ameisensäure als korrosionsinhibierendes Mittel.

Die in Frage kommenden zirkoniumarmen Werkstoffklassen sind deutlich billiger als die im Stand der Technik empfohlenen Werkstoffe, die einen hohen Anteil an Zirkonium aufweisen. Somit sind die Baukosten einer Anlage zur Herstellung von wasserfreier bzw. weitgehend wasserfreier Ameisensäure vergleichsweise günstig.

In der Regel sind zumindest die Oberflächen der Einrichtungen ii), iii), iv), v) und/oder vi), die mit Ameisensäure und dem Extraktionsmittel kontaktieren, aus dem zirkoniumarmen Werkstoff konstruiert - eine Oberfläche soll in diesem Zusammenhang als äußere dünne Schicht, bevorzugt von ca. 1 mm Dicke, verstanden werden.

Meist kontaktieren genannte Oberflächen mit Medien, die mindestens 1 % Ameisensäure und mindestens 1 %, bevorzugt 5 %, Extraktionsmittel enthalten.

In den Anlagenteilen, die teilweise oder vollständig aus den vorstehend genannten zirkoniumannen Werkstoffen konstruiert sind, werden Temperaturen bis maximal ca. 190°C und Drücke bis maximal ca. 3 bar erreichl Auch unter diesen hohen Temperaturen und Drücken sind diese Werkstoffe bei Anwesenheit des Extraktionsmittels als Konstruktionsmaterial geeignet.

In einer bevorzugten Ausfuhrungsform sind einer solchen Anlage die Einrichtungen iii) und iv) in einer einzigen Destillationseinrichtung angeordnet. Letztere ist in der Regel als Kolonne ansgebildet.

Als Extraktionsmittel werden bevorzugt N,N-Di-n-butylformamid, N,N-Di-n-butylacetamid, N-Methyl-N-2-heptylformamid, N-n-Butyl-N-2-ethylhexylformamid, N-n-Butyl-N-cyclohexylformamid und/oder N-Ethylformanilid eingesetzt.

Die Temperaturen betragen während des Verfahrens im Mittel, d. h. im Mittel über alle Stufen α) bis γ) 100°C. Insbesondere beträgt die durchschnittliche Temperatur pro Verfahrensstufe α) bis γ) 100°C.

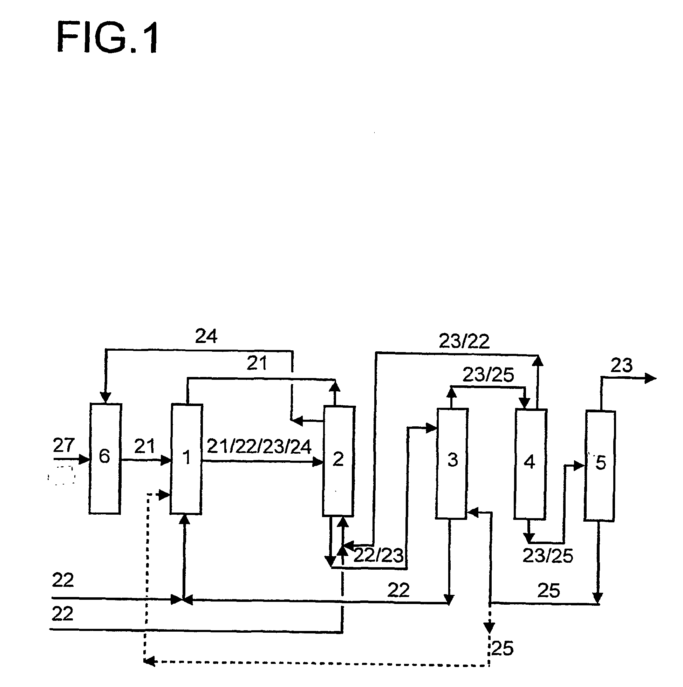

Die anliegende Zeichnung zeigt
- in Fig. 1: ein Schema einer Anlage zur Herstellung von wasserfreier bzw. weitgehend wasserfreier Ameisensäure,
- in Fig. 2: ein Schema einer Anlage zur Herstellung von wasserfreier bzw. weitgehend wasserfreier Ameisensäure, wobei die Destillationseinrichtung zur Durchführung der Stufe β) und die Destillationseinrichtung zur Durchführung der Stufe δ) in einer einzigen Destillationseinrichtung angeordnet sind.

Die über, unter bzw. neben den Pfeilen eingetragenen Bezugszeichen geben diejenigen Komponenten an, die im allgemeinen in den jeweiligen Strömen einen hohen Anteil bzw. den Hauptanteil aufweisen. Da die Anteile der Komponenten in den Strömen variieren können, sollten diese Bezugszeichen nur als Richtwert zur Orientierung dienen. Bezugszeichen 21 steht für Methylformiat, 22 für Wasser, 23 für Ameisensäure, 24 für Methanol, 25 für Extraktionsmittel und 27 für Kohlenmonoxid. Es ist aufgezeigt, daß Methylformiat in einem Synthesereaktor 6 hergestellt, die Hydrolyse des Methylformiats in einem Hydrolysereaktor 1 durchgeführt und die Durchführung der Stufe β) in einer Destillationseinrichtung 2 vorgenommen wird, die Extraktion in einer Extraktionseinrichtung 3 erfolgt, die Durchführung der Stufe δ) in einer Destillationseinrichtung 4 erfolgt und Stufe φ) in einer Destillationseinrichtung 5 vorgenommen wird.

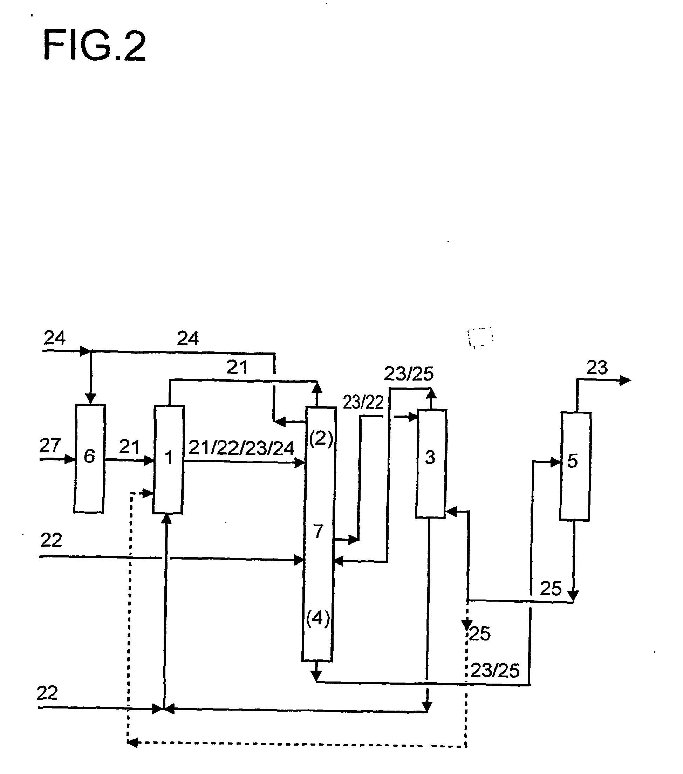

In Fig. 2 sind die Destillationseinrichtungen 2; 4 in einer einzigen Destillationseinrichtung 7 angeordnet.

Im folgenden soll die vorliegende Erfindung anhand eines Ausführungsbeispiels näher erläutert werden.

### Beispiel

Als Extraktionsmittel/Anitikorrosionsmittel wird N,N-Di-n-butylformamid eingesetzt. Die untersuchten Werkstoffe werden in einem Autoklaven dem korrosiven Medium ausgesetzt. Der Systemdruck beträgt 3 bar und die Probenverweilzeit 10 Tage. Die entsprechenden Werkstoffe werden hinsichtlich ihrer Beständigkeit in sieben verschiedenen Medien untersucht. Die Temperaturen bzw. die Zusammensetzungen der Gemische geben ungefähr die Bedingungen an, die in dem Verfahren zur Herstellung von wasserfreier bzw. weitgehend wasserfreier Ameisensäure vorliegen. Die Versuchsbedingungen sind in der nachstehenden Tabelle aufgeführt.

**Tabelle 9a**

| | | | | | | |
|---|---|---|---|---|---|---|
| **Komponenten** | **Gemisch 1** (wäßrige Phase) | **Gemisch 2** (organische Phase) | **Gemisch 3** (wäßrige Phase mit weniger AS) | **Gemisch 4** (organische Phase mit weniger AS) | **Gemisch 5** (Konzentrierte AS in DBF, hohe Temperatur) | **Gemisch 6** (ohne DBF) |
| AS Gew.-% | 20 | 16 | 5 | 5 | 12 | 85 |
| DBF Gew.-% | 2 | 72 | 2 | 83 | 87 | - |
| W Gew.-% | 78 | 12 | 93 | 12 | 1 | 15 |
| Temperatur °C | 110 | 110 | 110 | 110 | 160 | 150 |

| | | | | | | |
|---|---|---|---|---|---|---|
| AS = Ameisensäure, DBF = N,N-Di-n-butylformamid, W = Wasser | | | | | | |

Die Korrosionsuntersuchungen führen zu folgenden Ergebnissen:

**Tabelle 9b**

| **Gemisch** | 1 | 2 | 3 | 4 | 5 | 6 |
|---|---|---|---|---|---|---|
| **Werkstoffnummer** | | | | | | |
| Klasse I: 3.7235 (DIN 17851) | + | + | + | + | + | - |
| Klasse II: 2.4856 (DIN 17744, EN 10095) und 2.4819 (DIN 17744) | + | + | + | + | - | - |
| Klasse III: 1.4439 (EN 10088-1-2-3, DIN 17440, DIN 17441) | - | - | + | + | - | - |

In vorstehender Tabelle wird ein Werkstoff als beständig (+) bezeichnet, wenn er Korrosionsraten unter 0,1 mm/Jahr aufweist - anderenfalls wird der Werkstoff mit (-) bezeichnet.

Die in der vorstehenden Tabelle aufgeführten Ergebnisse zeigen, daß Werkstoffe aller drei Klassen I bis III in erfindungsgrmäße Extraktionsmittel/Antikorrosionsmittel enthaltenden und wäßrige Ameisensäure aufweisenden Gemischen beständig sind. Es wird auch deutlich, daß ohne den Einsatz des Extraktionsmittels/Antikorrosionsmittels sich die getesteten Werkstoffe weniger gut eignen.

## Patentansprüche

1. Verfahren zur Gewinnung von wasserfreier oder weitgehend wasserfreier Ameisensäure, bei dem man in einer Anlage
α) Methylformiat der Hydrolyse unterwirft,
β) vom erhaltenen Hydrolysegemisch Methanol sowie überschüssiges Methylformiat abdestilliert,
χ) das Ameisensäure und Wasser enthaltende Sumpfprodukt der Destillation (β) in einer Flüssig-flüssig-Extraktion mit einem hauptsächlich die Ameisensäure aufnehmenden Extraktionsmittel extrahiert und dabei als Extraktionsmittel ein Carbonsäureamid der allgemeinen Formel I 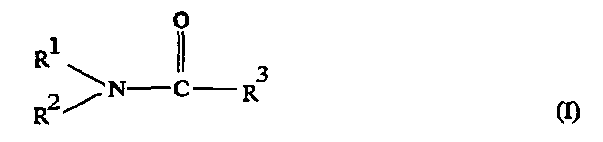 einsetzt, in der die Reste R¹ und R² Alkyl-, Cycloalkyl- Aryl- oder Aralkylgruppen bedeuten oder R¹ und R² gemeinsam zusammen mit dem N-Atom einen heterocyclischen 5- oder 6-Ring ausbilden und in der nur einer der Reste eine Arylgruppe ist und in der R³ für Wasserstoff oder eine C₁₋₄-Alkylgruppe steht,
δ) die hierbei erhaltene Ameisensäure, Extraktionsmittel und eine Teilmenge des Wassers aufweisende Extraktphase einer Destillation unterwirft,
ε) das bei dieser Destillation erhaltene Wasser und eine Teilmenge der Ameisensäure enthaltende Kopfprodukt in den unteren Teil der Destillationseinrichtung zur Durchführung der Stufe β) zurückführt,
φ) das vorwiegend Extraktionsmittel und Ameisenäure enthaltende Sumpfprodukt der Destillationsstufe ε) destillativ in wasserfreie oder weitgehend wasserfreie Ameisensäure und das Extraktionsmittel auftrennt und
γ) das die Stufe φ) verlassende Extraktionsmittel in den Verfahrensgang zurückfuhit,
**dadurch gekennzeichnet, daß** ein oder mehrere Anlagenteile, die mit Ameisensäure und mit Extraktionsmittel kontaktieren, teilweise oder vollständig aus einem zirkoniumarmen Werkstoff konstruiert sind, der ausgewählt ist aus der Gruppe der Werkstoffklassen Titan-Palladium-Legierungen, chrom-, molybdän- und/oder wolü=haltige Nickelbasiswerkstoffe und molybdänhaltige hochlegierte austenitische Chrom-Nickel-Sonderstähle, wobei
- die chrom-, molybdän- und/oder wolframhaltigen Nickelbasiswerkstoffe 14 bis 24 Gew.-% er, 8 bis 17 Gew.-% Mo und/oder 3 bis 5 Gew.-% W, maximal 25 Gew.-% sonstige Elemente und den Rest als Ni enthalten,
- die molybdänhaltigen hochlegierten austenitischen Chrom-Nickel-Soaderstähle 18 bis 30 Gew.-% er, 12 bis 40 Gew.-% Ni, 3 bis 7 Gew.-% Mo, maximal 3 Gew.-% Cu, maximal 0,05 Gew.-% C, maximal 25 Gew.-% sonstige Elemente und den Rest als Fe enthalten, und
- die Temperaturen im Verfahren im Mittel 75°C betragen,
und wobei die mit Ameisensäure und mit Extraktionsmittel kontaktierenden Anlagenteile als Reaktor zur Durchführung der Stufe α), als Destillationseinrichtung zur Durchführung der Stufe β), als Destillationseinrichtung zur Durchführung der Stufe δ), als Extraktionseinrichtung zur Durchführung der Stufe χ) und/oder als Destillationseinrichtung zur Durchführung der Stufe φ), vorliegen.

2. Verfahren nach Anspruch 1, **dadurch gekennzeichnet, daß** als Extraktionsmuittel N,N-Di-n-butylformamid, N,N-Di-n-butylacetamid, N-Methyl-N-2-heptylformamid, N-n-Butyl-N-2-ethylhexylformamid, N-n-Butyl-N-cyclohexylformamid und/oder N-Ethylformanilid eingesetzt wird.

## Revendications

1. Procédé de préparation d'acide formique anhydre ou presque parfaitement anhydre, dans lequel, dans une installation,
α) on soumet du formiate de méthyle à une hydrolyse,
β) on chasse par distillation du mélange d'hydrolyse ainsi obtenu le méthanol, ainsi que le formiate de méthyle en excès,
χ) on extrait, à l'aide d'un agent d'extraction qui absorbe essentiellement l'acide formique, le produit de fond, contenant de l'acide formique et de l'eau, de la distillation (β) dans le cadre d'une extraction liquide-liquide, et à cette occasion on utilise en tant qu'agent d'extraction un carboxamide de formule générale I 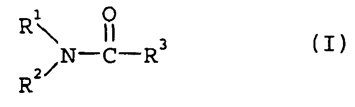 dans laquelle les radicaux R¹ et R² sont des groupes alkyle, cycloalkyle, aryle ou aralkyle, ou bien R¹ et R² avec l'atome d'azote, forment un noyau hétérocyclique à 5 ou 6 chaînons, et seul l'un des radicaux est un groupe aryle, R³ représentent un atome d'hydrogène ou un groupe alkyle en C₁-C₄,
δ) on soumet à une distillation la phase formant extrait, obtenue à cette occasion, et contenant de l'acide formique, l'agent d'extraction et une partie de l'eau,
ε) on renvoie le produit de tête, contenant l'eau obtenue lors de la distillation, ainsi qu'une partie de l'acide formique, dans la partie inférieure du dispositif de distillation servant à la mise en oeuvre de l'étape β),
φ) on sépare par distillation le produit de fond, contenant essentiellement l'agent d'extraction et de l'acide formique, de l'étape de distillation e), en acide formique anhydre ou presque parfaitement anhydre, et en l'agent d'extraction, et
γ) on renvoie dans le cycle d'opérations l'agent d'extraction sortant de l'étape φ),
**caractérisé en ce qu'**une ou plusieurs parties de l'installation, qui sont en contact avec l'acide formique et avec l'agent d'extraction, sont en partie ou en totalité construites en un matériau à faible teneur en zirconium, qui est choisi dans l'ensemble des classes de matériaux comprenant les alliages titane-palladium, les matériaux à base de nickel et contenant du chrome, du molybdène et/ou du tungstène, et les aciers austénitiques spéciaux au chrome-nickel hautement alliés et contenant du molybdène, où
- les matériaux à base de nickel, contenant du chrome, du molybdène et/ou du tungstène, contiennent 14 à 24 % en poids de Cr, 8 à 17 % en poids de Mo et/ou 3 à 5 % en poids de W, au maximum 25 % en poids d'autres éléments, le reste étant constitué de Ni,
- les aciers spéciaux austénitiques au chrome-nickel hautement alliés et contenant du molybdène contiennent 18 à 30 % en poids de Cr, 12 à 40 % en poids de Ni, 3 à 7 % en poids de Mo, au maximum 3 % en poids de Cu, au maximum 0,05 % en poids de C, au maximum 25 % en poids d'autres éléments, le reste étant constitué de Fe, et
- les températures, dans le procédé, sont en moyenne de 75°C,
les parties d'installation destinées à entrer en contact avec l'acide formique et avec l'agent d'extraction étant un réacteur pour la mise en oeuvre de l'étape α), un dispositif de distillation pour la mise en oeuvre de l'étape β), un dispositif de distillation pour la mise en oeuvre de l'étape δ), un dispositif d'extraction pour mettre en oeuvre l'étape χ), et/ou un dispositif de distillation pour mettre en oeuvre l'étape φ).

2. Procédé selon la revendication 1, **caractérisé en ce qu'**on utilise en tant qu'agent d'extraction le N,N-di-n-butylformamide, le N,N-di-n-butylacétamide, le N-méthyl-N-2-heptylformamide, le N-n-butyl-N-2-éthylhexylformamide, le N-n-butyl-N-cyclohexylformamide et/ou le N-éthyl-formanilide.

## Claims

1. A process for obtaining anhydrous or substantially anhydrous formic acid, in which, in a plant,
α) methyl formate is subjected to hydrolysis,
β) methanol and excess methyl formate are distilled off from the resultant hydrolysis mixture,
χ) the bottom product from distillation (3), comprising formic acid and water, is extracted in a liquid-liquid extraction with an extractant which principally takes up the formic acid, and the extractant employed here is a carboxamide of the general formula I 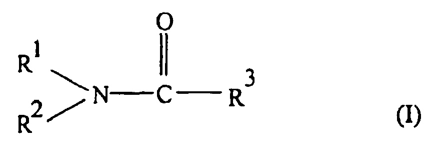 where the radicals R¹ and R² are alkyl, cycloalkyl, aryl or aralkyl groups, or R¹ and R² jointly, together with the N atom, form a heterocyclic 5- or 6-membered ring, and where only one of the radicals is an aryl group, and where R³ is hydrogen or a C₁-C₄-alkyl group,
δ) the resultant extract phase, comprising formic acid, extractant and some of the water, is subjected to distillation,
ε) the top product obtained in this distillation, which comprises water and some of the formic acid, is fed back into the lower part of the distillation device in step β),
φ) the bottom product from distillation step ε), which comprises predominantly extractant and formic acid, is separated by distillation into anhydrous or substantially anhydrous formic acid and the extractant, and
γ) the extractant leaving step φ) is fed back into the process,
**characterized in that** one or more plant parts which come into contact with formic acid and with extractant are constructed partly or completely of a low-zirconium material selected from the group consisting of the material classes titanium/palladium alloys, chromium-, molybdenum- and/or tungsten-containing nickel-based materials and molybdenum-containing, highly alloyed, austenitic chromium/nickel special steels, wherein
- the chromium-, molybdenum- and/or tungsten-containing nickel-based materials contain from 14% to 24% by weight of Cr, from 8% to 17% by weight of Mo and/or from 3% to 5% by weight of W, not more than 25% by weight of other elements and the remainder as Ni,
- the molybdenum-containing highly alloyed austenitic chromium/nickel special steels contain from 18% to 30% by weight of Cr, from 12% to 40% by weight of Ni, from 3% to 7% by weight of Mo, not more than 3% by weight of Cu, not more than 0.05% by weight of C, not more than 25% by weight of other elements and the remainder as Fe, and
- the temperatures in the process are 75°C on average,
and wherein the plant parts which come into contact with formic acid and with extractant are in the form of the reactor for carrying out step α), the distillation device for carrying out step β), the distillation device for carrying out step δ), the extraction device for carrying out step χ) and/or the distillation device for carrying out step φ).

2. The process according to claim 1, **characterized in that** the extractant employed is N,N-di-n-butylformamide, N,N-di-n-butylacetamide, N-methyl-N-2-ethylheptylformamide, N-n-butyl-N-2-ethylhexylformamide, N-n-butyl-N-cyclohexylformamide and/or N-ethylformanilide.
